**Europäisches Patentamt**

**European Patent Office**

**Office européen des brevets**

(19)

(11) **EP 1 674 862 A1**

(12) **EUROPEAN PATENT APPLICATION**

(43) Date of publication:
**28.06.2006 Bulletin 2006/26**

(51) Int Cl.:
***G01N 29/07*** (2006.01)

(21) Application number: **05076585.8**

(22) Date of filing: **12.07.2005**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK YU** | (71) Applicant: **Gamesa Desarrollos Aeronauticos, S.A.**<br>**01510 Miñano (ES)** |
| (30) Priority: **22.12.2004 ES 200403062** | (72) Inventors:<br>• **Peña Macias, Julio/Gamesa Desarrollos Aeronáuticos**<br>**01510 Miñano (Alava) (ES)**<br>• **Kawiecki, Gregorio/Gamesa Desarrollos Aeronáuticos**<br>**01510 Miñano (Alava) (ES)** |

(54) **Composite materials ultrasonic cure monitoring system and method**

(57)     Composite materials cure monitoring system and method based on the use of piezotransducers that can be activated by external signals and that, as a result of this activation, can generate elastic waves that can be detected by same, or distinct, piezotransducers distributed over the structure. The received signals are, then, processed by a dedicated acquisition and processing system with the objective to control the composite material cure process.

**FIG. 1**

**Description**

**Summary**

The present invention relates to a system and method for the monitorization of different resin and advanced composite materials cure processes, manufactured from small diameter fibers of different disposition, embedded in a resin matrix. Proposed system is of special application but not limited to curing processes of aeronautical structures.

**Background of the invention**

There are great number of patents related to monitoring composite material cure processes, employing different systems and procedures. **U.S. 4,891,591** patent, employs a system based on the variation of the electromagnetic field to measure the cure; this method has the disadvantage of being applicable only to materials having electric conductivity properties. **U.S. 4,921,415** patent deals with a system based on ultrasonic waves, appropriate for high temperature cure processes. **U.S. 5,009,104** patent also describes a system based on ultrasonic waves. **U.S. 5,436,565** patent describes a cure monitoring method based on the electric capacity measurement, applicable only to dielectric materials as epoxy resins. **U.S. 5,770,155** and **CN1350174** patents use fiber optic sensors; fiber optic sensors have the inconvenient to give information of the cure process up to resin gelation point, while the method related in this application is able to give information during all the cure process. **U.S.5,911,159** patent deals with monitoring resin parts by using guided acoustic waves, which implies the introduction of an acoustic wave conductor element to measure the propagation velocity of these waves through the resin piece. Finally **U.S.6,675,112 B1** patent describes the cure monitoring employing the response of the system to excitations based on very low frequency pressure waves; the method described in this application employs excitations of a higher frequency to obtain a better answer of the different vibration modes or very short electric waves to measure "time of flight" of the wave, so that, obtaining the material stiffness during the cure process.

**Description of the invention**

Composite materials have been significantly improved over last decade because they made it possible to produce lightweight, yet stiff structures with custom-tailored directional characteristics. One of the most critical phases of the manufacture process is the cure of the resin acting as matrix. Cure problems can lead to scrapping the manufactured part. During the cure process, the part is under a combination of temperature and pressure conditions, generally established by the raw material manufacturer, based on experimental data, and designed achieve correct matrix solidification.
However, standard cure processes are based on simple laboratory specimens and cannot be optimized for very complex geometry parts. Besides, in areas of large thickness of the part to be cured, high temperature gradients leading to a non-nominal cure can be produced. In a similar manner, an incorrect distribution of the heat sources can lead to an incomplete resin cure or, on the contrary, to an overcuring due to the high temperatures.
That is why, a system allowing to control in a real-time the cure process will help reducing the number of defects in the final part and/or to improve the cure cycle, minimizing the manufacturing time and, therefore, reducing costs. The system and method proposed in this document makes it possible to avoid the previously mentioned difficulties and to improve the composite material part manufacturing process, cutting down its cost due to the smaller proportion of scrap parts and due to the reduction on the manufacturing time, for the following reasons:

    1. There are no problems related to cure process temperature as sensors are able to give information during the entire cure process.
    2. This method is applicable both to resin and to different direction fiber laminates monitoring.
    3. It is not necessary to introduce a wave conductor element (U.S. 5,911,159) as, in the proposed system, waves are propagated along the manufactured part.
    4. As there is no dependence on the electromagnetic properties (U.S. 5,009,104) nor the electric capability (U.S. 5,436,565), there is no limitation on the manufactured part materials

**Brief description of the drawings.**

The figures included on this document help to explain the invention, supporting in a graphic way, the following description.

    Figure 1 represents a sketch of a possible lay-out of the piezotransducers in a composite material panel to be cured.

    Figure 2 shows an example of the variation of the answer in frequency against time, around a natural frequency of the composite material panel to be cured.

Figure 3 represents the variation on time of the natural frequency of Figure 2 (circle line) and its comparison against the experimental value (solid line).

Figure 4 shows an example of the evolution of the curing compressibility modulus of one panel of carbon fiber and epoxy resin against cure time (hours: minute)

## Description of the preferred embodiment

Method and system for cure monitoring related in this document is applicable for the different manufacturing process existing nowadays, including;

- Vacuum bag curing
- Oven curing
- Autoclave curing
- Compression moulding
- "Integrally heated tooling"

System involved in this patent comprises;

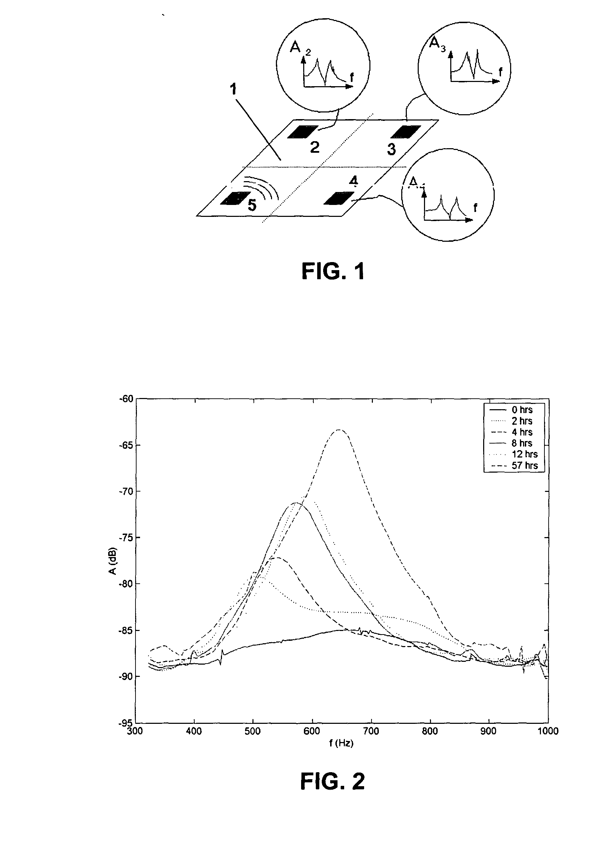

- A network of piezoelectric materials distributed over the part to be monitoring (1) as represented on Figure 1, made by, at least, one piezoelectric element capable of acting both as actuator element (5) and sensor (2), (3) &(4). Distribution of the piezoelectric elements will be so that covers the areas of the part to be cured considered critical and/or of special interest for the cure process. Such piezoelectric elements will be embedded in the part to be cured. For embedded it will understand to be placed between the laminates forming the laminate of the part (when talking about a composite material part) or totally surrounded by resin (in case of part manufactured only by resin) or in contact with the external surface of the part (for both cases). Connexion between piezoelectric materials and signal actuation and register equipments (description bellow) shall be done by cables and/or by a wireless signal transmission system.
  In any form, the required equipment will be installed (not described in this document) in the part, mould, oven or in the autoclave, to support the cables and/or the wireless equipments that connect the piezoelectric elements to the signal actuation and reception equipments. Piezoelectric material will be selected so its Curie temperature being, at least, two times the maximum temperature of the cure cycle. Therefore, it may be chosen, v.gr. but not solely, lead titanate-zirconate (PZT) for curing temperatures up to 100-150 °C, and litium tantalate (LiTa03) or litium niobiate (LiNb03) for greater temperatures.

- An electric signal generation system able to generate both impulse-like signals and short duration constant frequency waveforms or chirps. In the event of impulse signals being used as excitation signal of the system, two piezoelectric elements, at the least, shall be utilized, one of them playing the role of actuator and the other one, or the other ones, acting as sensor, or sensors. Any of the piezoelectric transducers of the network may operate as actuator (making use of the commutation system described below) behaving the remainder of them as sensor. In the event of using the second type of excitation signal (waveforms), the selected frequency (in case of constant frequency signals) or range of frequencies (in case of chirp signals) may excite the principal vibration modes of the specimen; in the event of using waveforms, there will be only one piezoelectric element playing the role of emitter also, whereas the signal reception could be done with the same transducer or and/or with other piezoelements that belongs to the piezotransducer network. The monitoring system will be composed, not solely, by stand-alone waveform generator or computer-based waveform generator. The amplitude of the required excitation signal could be, depending on the dissipative characteristics of the part to monitor, between some volts and thousand of volts, and consequently it could be necessary to complement the hardware with a dynamic signal amplifier, able to lead the generated excitation signal to the required voltage level, without significant alterations of the frequency contents.

- An electric signals acquisition system, able to record the response of the piezoelements that have the role of sensors. Generally, this system would comprise a digital oscilloscope and/or a computer-based data acquisition system, although not solely. If the voltage level of the electric signal coming from the piezoelectric sensors were higher than the maximum allowed by the acquisition system, an active or passive voltage divider, with a conversion ratio constant or variable, will be added, in order to transform the received signal to a suitable level for the signal acquisition system.

- In the case of the system comprises more than one piezoelectric element, it will be needed to use a switching device to be able to select the piezoelements that act as actuators as well as the piezoelements that act as sensors. Such device could be operated by means of a computer or manually.

The embodiments of this invention comprise:

- The installation of a piezoceramic transducers network arranged on a composite material part to be cured (FIG. 1) in such way that it includes areas that are critical of interest during the curing process. The piezoelectric elements should be installed far enough one from the others that it would be possible to measure properly the "time of fight" of the signal, between the actuator piezoelement and the sensor piezoelement with an error lower than 5%. If the investigated specimen would have different properties depending on the direction, due to the specific arrangement of the fibre plies, the piezoelectric transducers will be placed in such fashion that some of the direct propagation paths of the elastic waves, that travel between the actuator and the sensor piezotransducers, coincide with some of these directions.
- The connection of those elements to the signal generation and acquisition system, in such way as described above.
- The generation, by means of a signal generator connected to the piezoelements that have the role of actuators, of impulsive like signal, of very short duration (some milliseconds), or monochromatic waveforms. In the last case, the frequency of the signal will be included between 100 Hz and 20 kHz, and will consist of 3 to 7 cycles. Additionally a Hamming windowing (or other type) can be applied to avoid leakage in the frequency response.
- The transmission of the elastic wave, generated by the actuator piezoelement, through the corresponding part to be cured.
- The synchronized recording of the direct signal received by the other or other piezoelements, acting as sensors, placed on the part being cured, to measure the time of flight of the elastic waves. Once the time of flight of the signal ($t_i$) is obtained, and guessing that the distance between piezoelements ($d_i$) is known, the wave propagation velocity ($C_i$) can be calculated as:

$$C_i = \frac{d_i}{t_i} \qquad\qquad \text{Ec. (1)}$$

The rigidity characteristics of the specimen for the direction i can be obtained applying the following equation:

$$\kappa_i = \rho C_i^2 \qquad\qquad \text{Ec. (2)}$$

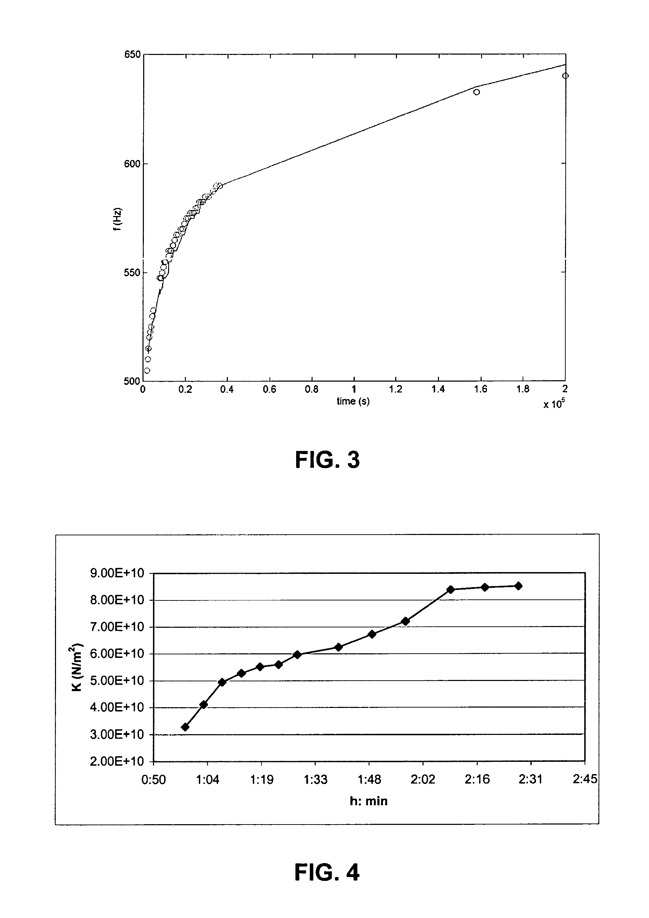

where, $\kappa i$ is the compressibility module and $p$ is the density of the material. The variation of this value versus time, as the presented in the FIG.4, compared with the theoretical expected evolution, gives an indication of the evolution of the curing process and when this has finished. By means of the measurement of this parameter in different places of the specimen, the uniform curing of the part can be guaranteed.

- The generation, by means of a signal generator connected to the actuator piezoelements, of chirps of short duration (some tenths of second) and a frequency range that includes one (the first) or some of the natural frequencies of the part to manufacture, that are susceptible to change during the curing development.
- The transmission of the elastic wave, generated by the actuator piezotransducer, through the part that is being cured.
- The synchronized record of the frequency response of the system at the locations of where piezotransducer sensors are located, as presented in the FIG. 2. In this situation, since the analytic determination of the eigenfrequencies of the set composed by the curing part and the mould is quite sophisticated, the pursuit of the curing process will be done by comparison between the variation in natural frequencies values and a representative variation in natural frequencies values versus time -previously obtained by means of experiments -, as shown in the FIG. 3. This comparison will allow to guarantee the properly development of the curing process and the introduction of variations of the curing process parameters, in order to obtain a proper cured part.

**Claims**

1. Composite materials cure monitoring system, resin or solid laminate, of those that use an electric wave generation system and an elastic wave reception system, **characterized by** a network of piezoelectric elements connected to the electric wave generation system and the elastic wave reception system, capable of generating and receiving elastic waves, being the piezoelectric elements in direct contact with the composite material and incorporating a commutation equipment capable of selecting the adequate piezoelectric element.

2. Monitoring system, according to first claim, **characterized by** the piezoelectric network being made up of at least one piezoelectric element capable of acting as emitter and receiver of elastic waves.

3. Monitoring system, according to first claim, **characterized by** the piezoelectric elements being either embedded or located on the surface of the composite part to monitor.

4. Monitoring system according to first claim, **characterized by** incorporating a commuting equipment that allows the selection of the piezoelectric element that act as actuator as well as that or those that behave as sensors.

5. Monitoring system, according to first claim, **characterized by** the connection between the electric wave generation system and the piezoelectric actuator is possible using either a cable system or a wireless system.

6. Composite materials curing monitoring method, **characterized by**;

    - the piezoelectric element distribution allows for measurements to be taken and it is coincident with a determinate direction.
    - piezoelectric material composition can withstand elevated temperature above composite material gelation point.
    - elastic waves transmit through the part
    - impulsive-like signals and short duration constant frequency waveforms or chirps are generated through the electric signal generation system.

7. Monitoring method, according to claim 6, **characterized by** the distribution of piezoelectric elements allowing for the measurement of the fly time of the wave between the emitter piezoelectric and the receiver.

8. Monitoring method, according to claim 6, **characterized by** the distribution of piezoelectric elements in the part to be monitored, when it is made of fiber fabric plies, is such, that the direction that joins the piezoelectrics matches one of the directions of the laminate fibers in the part to be manufactured.

9. Monitoring method, according to claim 6, **characterized by** the material the piezoelectric element is made of, having a Curie temperature at least twice as high as the maximum temperature to be reached within the cure cycle of the part to be monitored.

10. Monitoring method, according to claim 6, **characterized by** the elastic waves that travel between the piezoelectric elements being transmitted along the part to be monitored without the need of transmitting elements

11. Monitoring method, according to claim 6, **characterized by** the electric signal generation system being capable of generating both impulse-like signals and short duration constant frequency waveforms or chirps.

12. Monitoring method, according to claim 6, **characterized by** the fact that, if the generated electric signal is of monochromatic or variable frequency wave type, the employed frequencies are such that excites the monitored part in any of its fundamental modes of vibration.

13. Monitoring method, according to claim 6, **characterized by** the receiving system being capable of registering and storing the elastic signals of the piezoelectric receivers.

14. Monitoring method, according to claim 6, **characterized by**, if the generated electric signal being of the impulsive type, the presence of at least two piezoelectrics becomes necessary, one acting as emitter and the other as receiver.

15. Monitoring method, according to claim 6, **characterized by** if the generated electric wave being of chirp type, the presence of one piezoelectric element capable of acting as emitter and receiver is necessary.

**16.** Monitoring method, according to claim 6, **characterized by** the amplitude of the generated electrical signal being bigger than 5 volts and smaller than 1000 volts.

**FIG. 1**

**FIG. 2**

# FIG. 3

# FIG. 4

**EP 1 674 862 A1**

European Patent Office

# EUROPEAN SEARCH REPORT

Application Number

EP 05 07 6585

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | WO 01/39253 A (THE BOARD OF TRUSTEES OF THE LELAND STANFORD JUNIOR UNIVERSITY) 31 May 2001 (2001-05-31)<br>* abstract; claims 1,5,15; figures 1A-5B *<br>* page 8, paragraph 2 - page 14, paragraph 4 * | 1-6,8-18<br><br>7 | INV.<br>G01N29/07<br>G01N29/22<br>G01N29/34<br>G01N33/44<br>B29C35/02 |
| X<br><br>Y | EP 1 008 848 A (BAYER CORPORATION) 14 June 2000 (2000-06-14)<br>* abstract; claims 1,5; figures 1-3 *<br>* paragraphs [0073] - [0076] * | 1,6<br><br>7 | ADD.<br>G01N3/00 |
| X<br><br>Y | US 5 181 421 A (KLINE ET AL) 26 January 1993 (1993-01-26)<br>* abstract; claims 1,6,13; figures 1-7 *<br>* column 3, line 59 - column 5, line 48 *<br>* column 39, line 53 - column 40, line 55 * | 1,6<br><br>7 | |
| D,X | US 4 921 415 A (THOMAS, III ET AL) 1 May 1990 (1990-05-01)<br>* abstract; claim 1; figures 1-3 *<br>* column 3, line 7 - column 8, line 65 * | 1,6 | |
| | | | TECHNICAL FIELDS SEARCHED (IPC) |
| A | KAWIECKI G ET AL: "Rosette piezotransducers for damage detection" SMART MATERIALS AND STRUCTURES, IOP PUBLISHING LTD., BRISTOL, GB, vol. 11, no. 2, 5 April 2002 (2002-04-05), pages 196-201, XP002371272 ISSN: 0964-1726<br>* the whole document * | 1,6 | G01N<br>B29C |

The present search report has been drawn up for all claims

| Place of search | Date of completion of the search | Examiner |
|---|---|---|
| Munich | 5 April 2006 | Uttenthaler, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...................................................................

& : member of the same patent family, corresponding document

EPO FORM 1503 03.82 (P04C01)

9

## ANNEX TO THE EUROPEAN SEARCH REPORT
## ON EUROPEAN PATENT APPLICATION NO.

EP 05 07 6585

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2006

| Patent document cited in search report | | Publication date | Patent family member(s) | | Publication date |
|---|---|---|---|---|---|
| WO 0139253 | A | 31-05-2001 | AU | 3437801 A | 04-06-2001 |
| | | | CA | 2360759 A1 | 31-05-2001 |
| | | | EP | 1185839 A2 | 13-03-2002 |
| | | | JP | 2003515730 T | 07-05-2003 |
| EP 1008848 | A | 14-06-2000 | AU | 766664 B2 | 23-10-2003 |
| | | | AU | 6315599 A | 15-06-2000 |
| | | | CA | 2291524 A1 | 08-06-2000 |
| | | | NZ | 501575 A | 30-11-2001 |
| | | | US | 6066284 A | 23-05-2000 |
| US 5181421 | A | 26-01-1993 | NONE | | |
| US 4921415 | A | 01-05-1990 | NONE | | |

EPO FORM P0459

For more details about this annex : see Official Journal of the European Patent Office, No. 12/82